(19) Europäisches Patentamt
European Patent Office
Office européen des brevets

(11) **EP 4 439 568 A1**

(12) **EUROPEAN PATENT APPLICATION**
published in accordance with Art. 153(4) EPC

(43) Date of publication:
**02.10.2024 Bulletin 2024/40**

(21) Application number: **23879956.3**

(22) Date of filing: **26.07.2023**

(51) International Patent Classification (IPC):
**G16C 20/20** (2019.01)     **G16C 20/70** (2019.01)
**H10K 85/00** (2023.01)

(52) Cooperative Patent Classification (CPC):
**G16C 20/20; G16C 20/70; H10K 85/00;
H10K 99/00**

(86) International application number:
**PCT/KR2023/010805**

(87) International publication number:
**WO 2024/085373 (25.04.2024 Gazette 2024/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.10.2022 KR 20220133170**

(71) Applicant: **LG Chem, Ltd.**
**Yeongdeungpo-gu
Seoul 07336 (KR)**

(72) Inventors:
• **KONG, Changsun**
  **Daejeon 34122 (KR)**

• **KANG, Sungkyoung**
  **Daejeon 34122 (KR)**
• **KANG, Eunhye**
  **Daejeon 34122 (KR)**
• **YOO, Seung Jun**
  **Daejeon 34122 (KR)**
• **LEE, Kyu Hwang**
  **Daejeon 34122 (KR)**
• **LEE, Jaein**
  **Daejeon 34122 (KR)**

(74) Representative: **Hoffmann Eitle**
**Patent- und Rechtsanwälte PartmbB
Arabellastraße 30
81925 München (DE)**

(54) **THERMAL STABILITY DETERMINING APPARATUS AND METHOD**

(57)     An apparatus and method for determining thermal stability according to embodiments and experimental examples of the present invention, can easily determine thermal stability of a target material without a separate experiment by inputting particle information constituting the target material to acquire a molecular structure, acquiring structural information from the molecular structure, and determining thermal stability of the target material using the structural information, and can provide expected effects of high speed, high accuracy, and low cost by enabling new molecular design and high-speed selection of candidate materials.

[Figure 2]

# Description

This application claims priority to and the benefit of Korean Patent Application No. 10-2022-0133170 filed in the Korean Intellectual Property Office on October 17, 2022, the entire contents of which are incorporated herein by reference.

[Technical Field]

The present invention relates to an apparatus and method for determining thermal stability, and more particularly, to an apparatus and method for determining whether a target material is thermally stable from molecular structure information on the target material.

[Background Art]

An organic light emitting diode (OLED) is a device that emits light by electroluminescence phenomenon without a separate light source.
More specifically, an organic light emitting diode (OLED) includes organic compound layers provided in the form of a thin film between an anode electrode and a cathode electrode. In other words, the organic light emitting diode (OLED) is provided such that multi-layer organic thin films are formed between the anode electrode and the cathode electrode.

In general, the multi-layer organic thin film includes a hole injection layer (HIL), a hole transport layer (HTL), an emission layer (EML), an electron transport layer (ETL), and an electron injection layer (EIL).

Accordingly, in the organic light emitting diode (OLED), when a driving voltage is applied between the anode electrode and the cathode electrode, holes that have passed through the hole transport layer (HTL) and electrons that have passed through the electron transport layer (ETL) are transported to the emission layer (EML) to form excitons, resulting in the emission layer (EML) to emit visible light.

In this case, when a material with low thermal stability is used in the multi-layer organic thin film, there may be problems such as stress generation due to high-temperature fluidity and material diffusion into adjacent pixel areas, which can cause performance degradation and lifetime reduction of the entire device.

In this regard, in the related art, to evaluate the thermal stability of a material, a method is used to observe the thermal stability in the process of thermal decomposition using indicators such as glass transition temperature (Tg), decomposition temperature (Td), and molecular weight change.

However, since the conventional method of determining thermal stability requires actual thin films to be manufactured and evaluated through experiments, there has been a problem of time consuming and high cost.

[Detailed Description of the Invention]

[Technical Problem]

An object of the present invention to solve the above problems is directed to providing an apparatus for determining thermal stability with high speed, high accuracy, and low cost.

Another object of the present invention to solve the above problems is directed to providing a method of determining thermal stability with high speed, high accuracy, and low cost.

[Technical Solution]

An apparatus for determining thermal stability, according to an embodiment of the present invention to achieve the object described above, the apparatus includes: a memory; and a processor executing at least one instruction in the memory, wherein the at least one instruction comprises: an instruction configured to acquire particle information constituting the target material; an instruction configured to acquire a molecular structure using the particle information; an instruction configured to acquire structural information from the molecular structure; and an instruction configured to determine thermal stability of the target material using the structural information.

Here, the instruction configured to acquire a molecular structure using the particle information may include an instruction configured to acquire an energy-optimized molecular structure using the particle information.

Meanwhile, the instruction configured to acquire the energy-optimized molecular structure using the particle information may include an instruction configured to acquire an energy-optimized molecular structure using any one of first principles, semi-empirical, or empirical methods, based on the particle information.

In addition, the particle information may include atom information and molecule information on the target material.

For example, the particle information may include a core moiety forming the target material and at least one functional group that is capable of being substituted to at least one derivative by bonding with the core moiety.

In addition, the structural information may include at least one of radius of gyration (Rg) or asphericity (As).

Meanwhile, the instruction configured to acquire the structural information from the molecular structure may include: an instruction configured to calculate a gyration tensor from the molecular structure; and an instruction configured to calculate at least one of radius of gyration or asphericity from the gyration tensor.

In this case, the gyration tensor may be a matrix that is configured with a mass and position vectors of atoms.

In addition, the instruction configured to calculate at least one of the radius of gyration or asphericity may include: an instruction configured to diagonalize the gyration tensor to be expressed in terms of eigenvalues L1, L2, and L3; an instruction configured to calculate the radius of gyration using the eigenvalues of the gyration tensor; and an instruction configured to calculate the asphericity using the eigenvalues of the gyration tensor.

Meanwhile, the instruction configured to determine thermal stability of the target material may include an instruction configured to determine thermal stability of the target material by comparing the structural information to a threshold value.

Here, the instruction configured to determine thermal stability of the target material by the comparing may include: an instruction configured to determine that thermal stability of the target material is high when the radius of gyration and asphericity exceed a first threshold and a second threshold, respectively; and an instruction configured to determine that thermal stability of the target material is low when the radius of gyration and asphericity are equal to or less than the first threshold and the second threshold, respectively.

In addition, the instruction configured to determine thermal stability of the target material may include an instruction configured to determine thermal stability of the target material by inputting the structural information to a pre-trained learning model.

In this case, the pre-trained learning model may be a machine learning model using at least one experimental data according to a thermal stability experiment as training data.

In addition, the instruction configured to determine thermal stability of the target material may further include an instruction configured to store result data of the determination of thermal stability in a database.

In addition, the instruction configured to determine thermal stability of the target material may further include an instruction configured to retrain the learning model using the at least one result data stored in the database.

Meanwhile, the target material may be applied to an organic thin film layer of an organic light emitting diode (OLED) .

A method of determining thermal stability, according to another embodiment of the present invention to achieve another object described above, which is performed in the apparatus for determining thermal stability, the method includes: acquiring particle information constituting a target material; acquiring a molecular structure using the particle information; acquiring structural information from the molecular structure; and determining thermal stability of the target material using the structural information.

Here, the acquiring of the molecular structure using the particle information may include acquiring an energy-optimized molecular structure using the particle information.

Meanwhile, the acquiring of the energy-optimized molecular structure using the particle information may include acquiring an energy-optimized molecular structure using any one of first principles, semi-empirical, or empirical methods, based on the particle information.

In addition, the particle information may include atom information and molecule information on the target material.

For example, the particle information may include a core moiety forming the target material and at least one functional group that is capable of being substituted to at least one derivative by bonding with the core moiety.

Meanwhile, the structural information may include at least one of radius of gyration (Rg) or asphericity (As).

In addition, the acquiring of the structural information from the molecular structure may include: calculating a gyration tensor from the molecular structure; and calculating the structural information including at least one of radius of gyration or asphericity from the gyration tensor.

In this case, the gyration tensor may be a matrix that is configured with a mass and position vectors of atoms.

In addition, the calculating of the structural information may include: diagonalizing the gyration tensor to be expressed in terms of eigenvalues L1, L2, and L3; calculating the radius of gyration using the eigenvalues of the gyration tensor; and calculating the asphericity using the eigenvalues of the gyration tensor.

Meanwhile, the determining of thermal stability of the target material may include determining thermal stability of the target material by comparing the structural information to a threshold value.

Here, the determining of thermal stability of the target material by the comparing may include: determining that thermal stability of the target material is high when the radius of gyration and asphericity exceed a first threshold and a second threshold, respectively; and determining that thermal stability of the target material is low when the radius of gyration and asphericity are equal to or less than the first threshold and the second threshold, respectively.

In addition, the determining of thermal stability of the target material may include determining thermal stability of the target material by inputting the structural information to a pre-trained learning model.

In this case, the pre-trained learning model may be a machine learning model using at least one experimental data according to a thermal stability experiment as training data.

In addition, the determining of thermal stability of the target material may further include storing result data of the determination of thermal stability in a database.

In addition, the determining of thermal stability of the target material may further include retraining the learning model using the at least one result data stored in the database.

Meanwhile, the target material may be applied to an organic thin film layer of an organic light emitting diode (OLED).

[Advantageous Effects]

An apparatus and method for determining thermal stability according to embodiments and experimental examples of the present invention, can easily determine thermal stability of a target material without a separate experiment by inputting particle information constituting the target material to acquire a molecular structure, acquiring structural information from the molecular structure, and determining thermal stability of the target material using the structural information, and can provide expected effects of high speed, high accuracy, and low cost by enabling new molecular design and high-speed selection of candidate materials.

[Brief Description of Drawings]

FIG. 1 is a block diagram of an apparatus for determining thermal stability according to an embodiment of the present invention.

FIG. 2 is a flowchart of a method of determining thermal stability performed by a processor operation of the apparatus for determining thermal stability according to an embodiment of the present invention.

FIG. 3 is a molecular structure image for describing particle information on a target material according to an embodiment of the present invention.

FIG. 4 is a flowchart for describing a step of acquiring structural information in the method of determining thermal stability, according to an embodiment of the present invention.

FIG. 5 is a flowchart for describing a method of determining thermal stability using a pre-trained learning model, in the method of determining thermal stability according to an embodiment of the present invention.

FIG. 6 is an image of an organic thin film deposition for a thermal stability experiment according to a Comparative Example of the present invention.

FIG. 7 is a graph of thermal stability experiment results by temperature according to radius of gyration and asphericity of each of a plurality of target materials according to a first Experimental Example of the present invention.

FIG. 8 is an image representing bonding position information of particle information on a target material for verifying thermal stability according to a second Experimental Example of the present invention.

FIG. 9 is an image illustrating thermal stability experiment results of target materials by adjusting bonding positions of functional groups according to the second Experimental Example of the present invention.

FIG. 10 is a graph of thermal stability distribution according to radius of gyration of a plurality of organic materials according to the second Experimental Example of the present invention.

FIG. 11 is a graph of thermal stability distribution according to asphericity of a plurality of organic materials according to the second Experimental Example of the present invention.

<Explanation of Reference Numerals and Symbols>

| | |
|---|---|
| 100: | MEMORY |
| 200: | PROCESSOR |
| 300: | TRANSMITTING/RECEIVING DEVICE |
| 400: | INPUT INTERFACE DEVICE |
| 500: | OUTPUT INTERFACE DEVICE |
| 600: | STORAGE DEVICE |
| 700: | BUS |

[Best Mode]

The present invention may be variously changed and may have various embodiments, and specific embodiments illustrated in the drawings will be described in the detailed description of the invention in detail. However, the description of the exemplary embodiments is not intended to limit the present invention to the particular exemplary embodiments, but it should be understood that the present invention is to cover all modifications, equivalents and alternatives falling within the spirit and technical scope of the present invention. In the description of the drawings, similar reference numerals are used for similar constituent elements.

The terms such as "first," "second," "A," "B," and other numerical terms may be used herein only to describe various elements, but these elements should not be limited by these terms. These terms are used only to distinguish one constituent element from another constituent element. For example, a first component may be named a second component, and similarly, the second component may also be named the first component, without departing from the scope of the present invention. The term "and/or" includes any and all combinations of a plurality of the related and listed items.

When one constituent element is described as being "coupled" or "connected" to another constituent element, it should be understood that one constituent element can be coupled or connected directly to another constituent element, and an intervening constituent element can also be present between the constituent elements. When one constituent element is described as being "coupled directly to" or "connected directly to" another constituent element, it should be understood that no intervening constituent element is present between the constituent elements.

The terminology used herein is used for the purpose of describing particular embodiments only and is not intended to limit the present invention. Singular expressions include plural expressions unless clearly described as different meanings in the context. The terms "comprises," "comprising," "includes," "including," "containing," "has," "having" or other variations thereof are inclusive and therefore specify the presence of stated features, integers, steps, operations, elements, and/or components, but do not preclude the presence or addition of one or more other features, integers, steps, operations, elements, components, and/or groups thereof.

Unless otherwise defined, all terms used herein, including technical or scientific terms, have the same meaning as commonly understood by those skilled in the art to which the present invention pertains. The terms such as those defined in a commonly used dictionary should be interpreted as having meanings consistent with meanings in the context of related technologies and should not be interpreted as ideal or excessively formal meanings unless explicitly defined in the present application.

Hereinafter, exemplary embodiments according to the present invention will be described in detail with reference to the accompanying drawings.

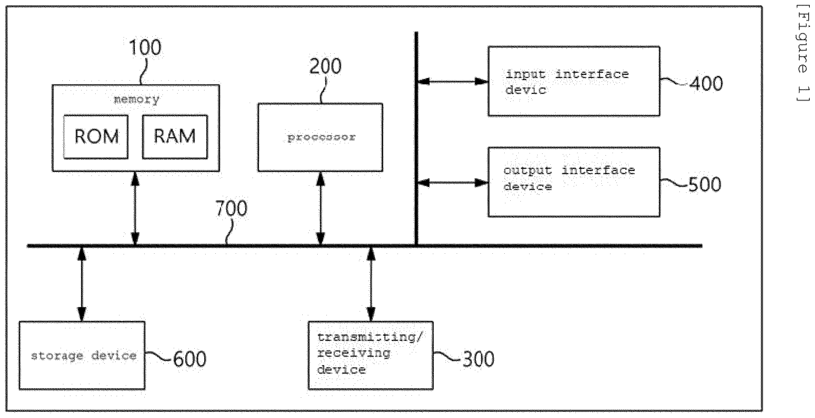

FIG. 1 is a block diagram of an apparatus for determining thermal stability according to an embodiment of the present invention.

With reference to FIG. 1, an apparatus for determining thermal stability according to an embodiment of the present invention may be an apparatus for determining thermal stability by acquiring structural information from particle information on a target material.

According to an embodiment, the apparatus for determining thermal stability may be used to select a material for an organic thin film layer of an organic light emitting diode (OLED). For example, the apparatus for determining thermal stability may generate a molecular structure from molecular information, which is particle information on an organic material input as a target material, and based on which, acquire structural information to identify whether the organic material is thermally stable. Accordingly, the apparatus for determining thermal stability may determine whether to select a material for the organic thin film layer of the organic light emitting diode (OLED) based on thermal stability information on the organic material.

Further describing the apparatus for determining thermal stability according to an embodiment of the present invention by hardware configuration, the apparatus for determining thermal stability may include a memory 100, a processor 200, a transmitting/receiving device 300, an input interface device 400, an output interface device 500, and a storage device 600.

According to an embodiment, each of the constituent elements 100, 200, 300, 400, 500, and 600 included in the apparatus for determining thermal stability may be connected by a bus 700 to communicate with each other.

The memory 100 and storage device 600 among the above configurations 100, 200, 300, 400, 500, and 600 may be configured as at least one of a volatile storage medium or a non-volatile storage medium. For example, the memory 100 and storage device 600 may be configured as at least one of a read-only memory (ROM) or a random access memory (RAM).

Among those, the memory 100 may include at least one instruction executed by the processor 200.

According to an embodiment, the at least one instruction may include an instruction to acquire particle information constituting a target material, an instruction to acquire a molecular structure using the particle information, an instruction to acquire structural information from the molecular structure, and an instruction to determine thermal stability of the target material using the structural information.

Here, the instruction to acquire a molecular structure using the particle information may include an instruction to acquire an energy-optimized molecular structure using the particle information.

Meanwhile, the instruction to acquire the molecular structure may include an instruction to acquire an energy-optimized molecular structure using any one of first principles, semi-empirical, or empirical methods, based on the particle information.

In addition, the particle information may include atom information and molecule information on the target material.

For example, the particle information may include a core moiety forming the target material and at least one functional group that is capable of being substituted to at least one derivative by bonding with the core moiety.

In addition, the structural information may include at least one of radius of gyration (Rg) or asphericity (As).

Meanwhile, the instruction to acquire structural information from the molecular structure may include an instruction to calculate a gyration tensor from the molecular structure information, and an instruction to calculate at least one of radius of gyration or asphericity from the gyration tensor.

In this case, the gyration tensor may be a matrix that is configured with a mass and position vectors of atoms.

In addition, the instruction to calculate at least one of the radius of gyration or asphericity may include an instruction to diagonalize the gyration tensor to be expressed in terms of eigenvalues L1, L2, and L3, an instruction to calculate the radius of gyration using the eigenvalues of the gyration tensor, and an instruction to calculate the asphericity using the eigenvalues of the gyration tensor.

Meanwhile, the instruction to determine thermal stability of the target material may include an instruction to determine thermal stability of the target material by comparing the structural information to a threshold value.

Here, the instruction to determine thermal stability of the target material may include an instruction to determine that thermal stability of the target material is high when the radius of gyration and asphericity exceed a first threshold and a second threshold, respectively, and an instruction to determine that thermal stability of the target material is low when the radius of gyration and asphericity are equal to or less than the first threshold and the second threshold, respectively.

In addition, the instruction to determine thermal stability of the target material may include an instruction to determine thermal stability of the target material by inputting the structural information to a pre-trained learning model.

In this case, the pre-trained learning model may be a machine learning model using at least one experimental data according to a thermal stability experiment as training data.

In addition, the instruction to determine thermal stability of the target material may further include an instruction to store result data of the determination of thermal stability in a database.

In addition, the instruction to determine thermal stability of the target material may further include an instruction to retrain the learning model using the at least one result data stored in the database.

Meanwhile, the target material may be applied to an organic thin film layer of an organic light emitting diode (OLED).

Meanwhile, the processor 200 may mean a central processing unit (CPU), a graphic processing unit (GPU), or an exclusive processor for executing the methods according to the embodiments of the present invention.

The processor 200 may execute at least one program instruction stored in the memory 100, as described above.

The apparatus for determining thermal stability according to an embodiment of the present invention has been described above. Hereinafter, a method of determining thermal stability performed by an operation of a processor in the apparatus for determining thermal stability will be described.

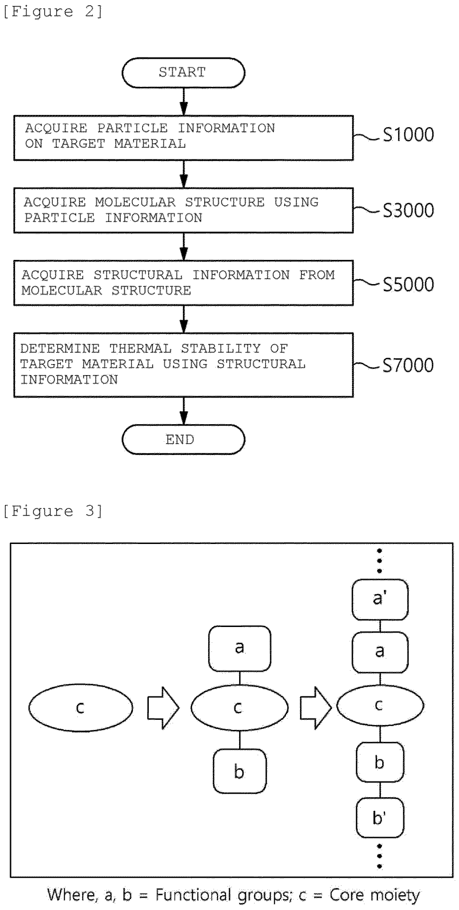

FIG. 2 is a flowchart of a method of determining thermal stability performed by a processor operation of the apparatus for determining thermal stability according to an embodiment of the present invention.

With reference to FIG. 2, the apparatus for determining thermal stability according to an embodiment of the present invention may acquire particle information of the target material (S1000). For example, the target material may be an organic material applied to a multilayer organic thin film layer of the organic light emitting diode (OLED). For example, the target material may be anthracene derivatives.

FIG. 3 is a molecular structure image for describing particle information on the target material according to an embodiment of the present invention.

With reference to FIG. 3, the apparatus for determining thermal stability may acquire particle information constituting the target material from a user.

The particle information may include atom information or molecule information. In other words, the particle information may be provided as atom information or molecule information, depending on the target material to be determined for thermal stability. For example, when the target material is an organic material, the user may input molecule information of the organic material in the apparatus for determining thermal stability.

According to an embodiment, the particle information may include a core moiety C forming the target material and at least one functional group a, a', b, and b' that is capable of being substituted to at least one derivative by bonding with the core moiety. For example, a core of the target material may be an anthracene molecule, and the at least one functional group may be at least one of benzene, naphthalene, phenanthrene, dibenzofuran, chrysene, pyrene, fluorene, benzo tetraphene, and benzo(a)pyrene, but is not limited to those disclosed herein.

In this case, a plurality of functional groups may be provided in extended states (a, a', a", ...) (b, b', b", ...) by chemical bonding. In this case, the plurality of functional groups may be provided by connecting molecules of the same or different structures, regardless of the structure.

According to an embodiment, the functional groups participating in the chemical bonding may be either electron-rich groups or electron-deficient groups.

According to another embodiment, the functional groups participating in the chemical bonding may be provided as a molecule that serves as an electron donor or an electron acceptor.

With reference back to FIG. 2, the apparatus for determining thermal stability may acquire a molecular structure using the particle information (S3000).

More specifically described according to an embodiment, the apparatus for determining thermal stability may acquire an energy-optimized molecular structure using at least one particle information. For example, the apparatus for determining thermal stability may acquire any one energy-optimized molecular structure based on the particle information, using any one of first principles, semi-empirical, or empirical methods. Here, the energy-optimized molecular structure may be defined as a stabilized molecular structure with a minimum energy close to the ground state.

In other words, the apparatus for determining thermal stability may generate at least one derivative that may be generated based on the at least one particle information. According to an embodiment, the apparatus for determining thermal stability may generate at least one derivative by adding at least one functional group to the core moiety.

According to another embodiment, the apparatus for determining thermal stability may generate at least one derivative by altering at least one functional group positioned on the core moiety.

According to still another embodiment, the apparatus for determining thermal stability may generate at least one derivative by removing at least one functional group positioned on the core moiety.

According to still another embodiment, the apparatus for determining thermal stability may generate at least one derivative by altering a chemical bonding position of at least one functional group positioned on the core moiety.

Thereafter, the apparatus for determining thermal stability may select a molecular structure of a derivative for which the energy is optimized among the at least one derivative acquired.

Then, the apparatus for determining thermal stability may acquire structural information from the selected molecular structure (S5000).

According to an embodiment, the structural information may include radius of gyration (Rg) and asphericity (As).

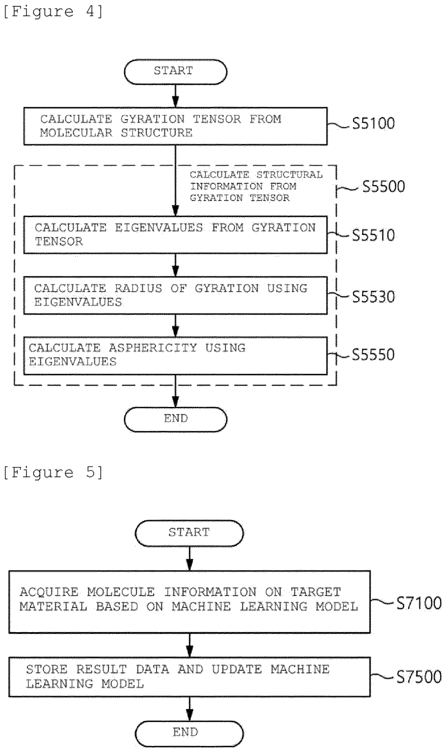

FIG. 4 is a flowchart for describing a step of acquiring structural information in the method of determining thermal stability, according to an embodiment of the present invention.

With reference to FIG. 4, the apparatus for determining thermal stability may calculate a gyration tensor from a molecular structure (S5100).

Here, the gyration tensor may be a physical quantity that mathematically represents the position, orientation, shape, movement, and transformation of a target material in a coordinate space.

According to an embodiment, the gyration tensor may be expressed in terms of a mass and position vectors of an each atom of a molecular structure, as shown in [Equation 1] below.

[Equation 1]

$$G = \sum_{j=1}^{n} \sum_{i=1}^{n} g_{ij} a_i a_j$$

G: Gyration tensor
$g_{ij}$: Mass of atom
$a_i$, $a_j$: Position vector

Then, the apparatus for determining thermal stability may convert the gyration tensor to a matrix form, as shown in [Equation 2] below.

[Equation 2]

$$G = \begin{bmatrix} G_{xx} & G_{xy} & G_{xz} \\ G_{yx} & G_{yy} & G_{yz} \\ G_{zx} & G_{zy} & G_{zz} \end{bmatrix}$$

G: Gyration tensor in matrix form

Then, the apparatus for determining thermal stability may calculate structural information including radius of gyration and asphericity from the gyration tensor (S5500).

More specifically, the gyration tensor matrix may be diagonalized, as shown in [Equation 3] below.

[Equation 3]

$$G^{diag} = \begin{bmatrix} L_1 & 0 & 0 \\ 0 & L_2 & 0 \\ 0 & 0 & L_3 \end{bmatrix}$$

$G^{diag}$ : Diagonalized gyration tensor
$L_1$, $L_2$, $L_3$: Eigenvalues of gyration tensor

Accordingly, the apparatus for determining thermal stability may calculate eigenvalues $L_1$, $L_2$, and $L_3$ of eigenvectors from the gyration tensor matrix (S5510).

Then, the apparatus for determining thermal stability may calculate radius of gyration using the eigenvalues $L_1$, $L_2$, and $L_3$ of the gyration tensor, as shown in [Equation 4] and [Equation 5] (S5530).

[Equation 4]

$$Rg^2 = L_1 + L_2 + L_3$$

Rg: Radius of gyration
$L_1$, $L_2$, $L_3$: Eigenvalues of gyration tensor

[Equation 5]

$$L_1 < L_2 < L_3$$

$L_1$, $L_2$, $L_3$: Eigenvalues of gyration tensor

In addition, the apparatus for determining thermal stability may calculate asphericity (As) using the eigenvalues $L_1$, $L_2$, and $L_3$ of the gyration tensor, as shown in [Equation 6] below (S5550).

[Equation 6]

$$As = I_3 - \frac{1}{2}(I_1 + I_2)$$

As: Asphericity

With reference back to FIG. 2, the apparatus for determining thermal stability may determine thermal stability of the target material based on the structural information (S7000).

According to an embodiment, the apparatus for determining thermal stability may determine thermal stability of the target material by comparing the structural information with a threshold value.

More specifically, the apparatus for determining thermal stability may determine thermal stability of the target material when radius of gyration is equal to or less than a first threshold value and asphericity is equal to or less than a second threshold value.

According to another embodiment, the apparatus for determining thermal stability may determine thermal stability of the target material by inputting the structural information into a pre-trained learning model.

FIG. 5 is a flowchart for describing a method of determining thermal stability using a pre-trained learning model, in the method of determining thermal stability according to an embodiment of the present invention.

With reference to FIG. 5, as described above, the apparatus for determining thermal stability may determine thermal stability of the target material by inputting structural information to the pre-trained learning model (S7100).

More specifically, the apparatus for determining thermal stability may output whether the target material is thermally stable by inputting at least one of radius of gyration or asphericity of the target material to the pre-trained learning model. For example, the apparatus for determining thermal stability may output whether the target material is thermally stable by inputting both radius of gyration and asphericity to the pre-trained learning model.

Here, the pre-trained learning model may be a machine learning model using at least one experimental data according to a thermal stability experiment as training data.

Then, the apparatus for determining thermal stability may store at least one result data acquired from the pre-trained learning model in a separate database, and update the learning model based on the result data (S7500).

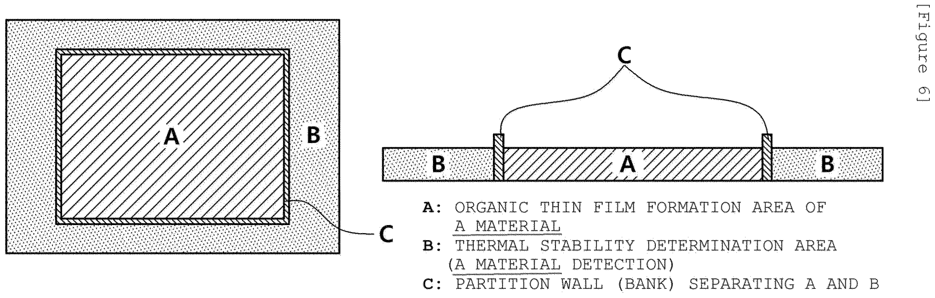

FIG. 6 is an image of an organic thin film deposition for a thermal stability experiment according to a Comparative Example of the present invention.

With reference to FIG. 6, in the thermal stability experiment, the target material may first be coated on a substrate to be formed into a thin film. For example, the target material may be coated on a substrate by a deposition process or a solution process to be formed into a thin film.

Here, the substrate may include a partition wall C surrounding a partial area A positioned at an upper portion of the substrate. Accordingly, in the thermal stability experiment, the target material may be coated and formed into a thin film only on the partial area A.

Here, the partition wall C is a configuration that physically separates and distinguishes the partial area A in the substrate and an outer area B excluding the partial area A. The target material cannot diffuse and invade between the two areas, but the composition of the target material may be detected in the outer area that is not coated with the target material due to evaporation or scattering by high temperature.

Accordingly, the thermal stability experiment may be conducted in a manner of gradually increasing a temperature of the thin film coated with the target material in the partial area A, and identifying whether the target material is detected in other areas except the partial area due to evaporation or scattering. In other words, with the thermal stability experiment, it is possible to determine whether there is thermal stability based on whether evaporation or scattering occurs in response to temperature.

Accordingly, based on at least one target material having been determined whether there is thermal stability by results of the thermal stability experiment, the learning model may be a model trained using structural information of the target material and the experimental results as training data. Here, the structural information may include at least one of radius of gyration or asphericity of the target material, as described above.

The apparatus and method for determining thermal stability according to an embodiment of the present invention have been described above.

Hereinafter, verification experiments of the apparatus for determining thermal stability according to Experimental Examples of the present invention will be described.

First verification experiment of apparatus for determining thermal stability

The apparatus for determining thermal stability according to an embodiment of the present invention was verified using the thermal stability experiment for training the learning model described above.

More specifically, for verification of the apparatus for determining thermal stability, at least one target material including a core moiety and functional groups was prepared. In this case, the at least one target material may be derivatives.

Then, a plurality of thin films with each of the target materials coated were prepared, as shown in the thermal stability experiment according to FIG. 6.

Then, the temperature of the films was gradually increased to a low temperature of 145°C or less, a medium temperature of 150°C, and a high temperature of 160°C or more, and the outer area of each film was examined for the detection of the corresponding target material.

Then, radius of gyration and asphericity were calculated for each of the target materials.

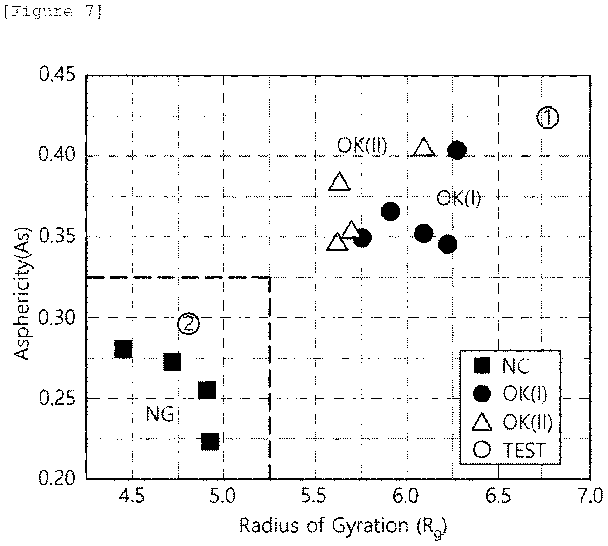

FIG. 7 is a graph of thermal stability experiment results by temperature according to radius of gyration and asphericity of each of a plurality of target materials according to the first Experimental Example of the present invention.

With reference to FIGS. 6 and 7, from the experimental results, it can be seen that when the temperature of the thin film is 150°C or more, the target materials detected in the outer area B on each substrate are concentrated in the first area ① in the graph of FIG. 7, where the horizontal axis represents information on radius of gyration and the vertical axis represents information on asphericity.

In addition, it can be seen that when the temperature of the thin film is 145°C or less, the target materials detected in the outer area B on each substrate are concentrated in the second area ② in the graph of FIG. 7.

Therefore, it can be seen that it is possible to determine thermal stability from the information on radius of gyration and asphericity of the target material.

According to an embodiment, it can be seen that when radius of gyration of the target material exceeds the first threshold value and asphericity exceeds the second threshold value, as shown in the first area ① in the graph of FIG. 7, thermal stability is high.

According to another embodiment, it can be seen that when radius of gyration of the target material is equal to or less than the first threshold value and asphericity is equal to or less than the second threshold value, as shown in the second area ② in the graph of FIG. 7, thermal stability is low.

**Second verification experiment of apparatus for determining thermal stability**

For the thermal stability experiment, a plurality of anthracene derivatives including an anthracene molecule as the core moiety and particle information on benzene, naphthalene, and dibenzofuran as the functional groups were prepared.

More specifically, as a plurality of anthracene derivatives, three derivatives were prepared in which bivalent phenylene group, bivalent naphthalene group, and monovalent dibenzofuranyl group were attached to bivalent anthracene core molecule at different positions.

Then, as in the first verification experiment, individual thin films with the above derivatives being the target material were prepared, and while the temperature of the thin films was gradually increased to a low temperature of 145°C or less, a medium temperature of 150°C, and a high temperature of 160°C or more, the outer area in each thin film was examined for the detection of the corresponding target material.

Then, radius of gyration and asphericity were calculated for each of the target materials.

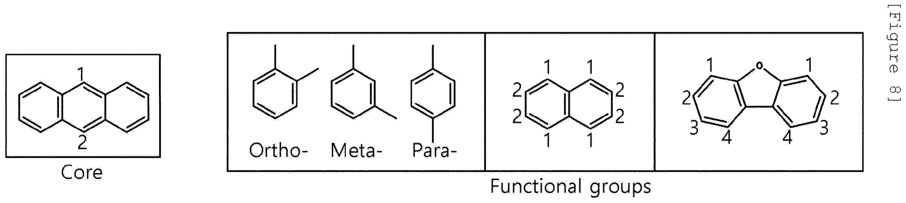

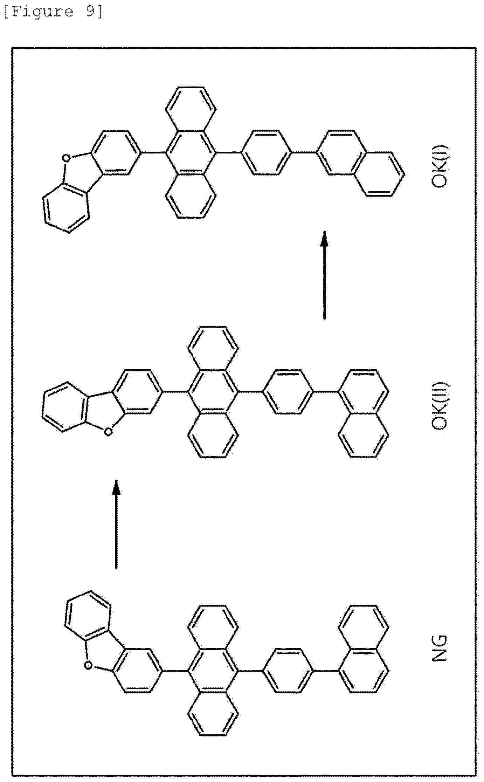

FIG. 8 is an image representing bonding position information of particle information on a target material for verifying thermal stability according to the second Experimental Example of the present invention, and FIG. 9 is an image illustrating thermal stability experiment results of target materials by adjusting bonding positions of functional groups.

With reference to FIGS. 8 and 9, the target materials are provided as first to third derivatives in which the bonding positions of the benzene, naphthalene, and dibenzofuran molecules are linked differently.

Here, the first derivative is a derivative in which, with respect to the anthracene molecule that is the core moiety, 1 of the anthracene molecule and 3 of the dibenzofuran molecule are bonded, and 2 of the anthracene molecule and 1 of the naphthalene molecule are bonded to para-position of benzene.

In addition, the second derivative is a derivative in which, with respect to the anthracene molecule that is the core moiety, 1 of the anthracene molecule and 2 of the dibenzofuran molecule are bonded, and 2 of the anthracene molecule and 1 of the naphthalene molecule are bonded to para-position of benzene.

Further, the third derivative is a derivative in which, with respect to the anthracene molecule that is the core moiety, 1 of the anthracene molecule and 3 of the dibenzofuran molecule are bonded, and 2 of the anthracene molecule and 2 of the naphthalene molecule are bonded to para-position of benzene.

In other words, the first and second derivatives are provided with different bonding positions of the anthracene molecule that is the core moiety and the dibenzofuran molecule that is the functional group.

In addition, the first derivative and the third derivative are provided with different bonding positions of the benzene linked to the anthracene molecule that is the core moiety and the naphthalene molecule that is the functional group.

Further, the second derivative and the third derivative are provided with different bonding positions of the dibenzofuran and naphthalene molecules that are functional groups linked to the anthracene molecule that is the core moiety.

In addition, through the thermal stability experiment, it was confirmed that the first derivative was evaporated or scattered at a temperature of 145°C or less, and thus was found to have low thermal stability (NG).

Meanwhile, it was confirmed that the second derivative and the third derivative were evaporated or scattered at temperatures of 165°C and 150°C or more, respectively, and thus were found to have high thermal stability (OK).

In other words, through the second experiment of the present invention, it can be seen that thermal stability can be improved by changing only the bonding positions of the functional groups.

Therefore, the apparatus and method for determining thermal stability according to embodiments of the present invention can determine thermal stability of a target material by acquiring particle information of the target material and measuring radius of gyration and asphericity according to bonding position of at least one molecule, without conducting a separate experiment.

Accordingly, the apparatus and method for determining thermal stability can also be useful in a technology for designing a molecular structure of a target material having thermal stability because the adjustment of the bonding position and bonding structure of the molecular components of the target material and thereby the thermal stability examination are facilitated.

In addition, the apparatus and method for determining thermal stability, for example, can be applied to any one electronic device including an organic light emitting diode (OLED) to examine thermal stability of a target material in an organic thin film layer of the organic light emitting diode, thereby determining a cause in the event of a quality anomaly in the electronic device, or can be used in conjunction with a device to monitor the lifetime of the electronic device.

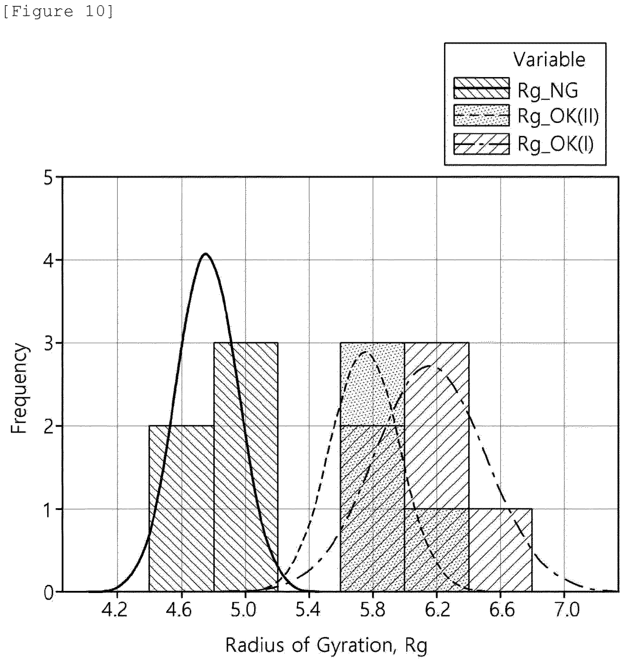

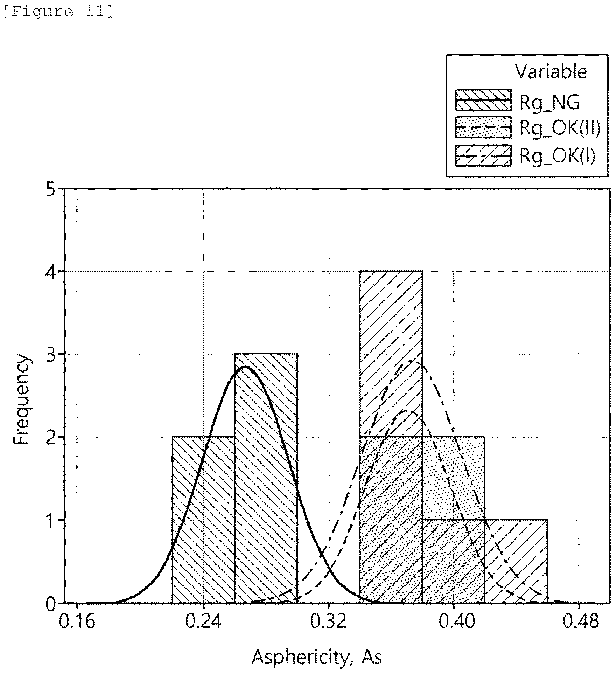

FIG. 10 is a graph of thermal stability distribution according to radius of gyration of a plurality of organisms according to the second Experimental Example of the present invention, and FIG. 11 is a graph of thermal stability distribution according to asphericity of a plurality of organisms.

With reference to FIGS. 10 and 11, thermal stability distribution according to radius of gyration and thermal stability distribution according to asphericity were measured for the first to third derivatives according to the second Experimental Example of the present invention.

As a result of interpreting the results of the second experiment of the first to third derivatives as thermal stability distribution with respect to radius of gyration, it can be seen that the lower thermal stability of the first to third derivatives is clearly distinguished, but it is difficult to distinguish the thermal stability at a high temperature.

In addition, as a result of interpreting the results of the second experiment of the first and third derivatives as thermal stability distribution with respect to asphericity, it can be seen that it is difficult to clearly distinguish thermal stability of the first and third derivatives in a high temperature area.

In other words, the apparatus and method for determining thermal stability according to an embodiment of the present invention can provide an apparatus and method for determining thermal stability with improved accuracy by reflecting all information on radius of gyration and asphericity as structural information from particle information of a target material.

The apparatus and method for determining thermal stability according to the embodiments and the Experimental Examples of the present invention have been described above.

An apparatus and method for determining thermal stability according to embodiments and Experimental Examples of the present invention, can easily determine thermal stability of a target material without a separate experiment by inputting particle information constituting the target material to acquire a molecular structure, acquiring structural information from the molecular structure, and determining thermal stability of the target material using the structural information, and can provide expected effects of high speed, high accuracy, and low cost by enabling new molecular design and high-speed selection of candidate materials.

The operation of the method according to the embodiments and Experimental Examples of the present invention may be implemented as a computer-readable program or code on a computer-readable recording medium. The computer-readable recording medium includes all kinds of storage devices for storing data readable by a computer system. In addition, the computer-readable recording medium may be distributed across a computer system that is networked, so that computer-readable program or code may be stored and executed in a distributed manner.

In addition, the computer-readable recording medium may include hardware devices that are specifically configured to store and execute program instructions, such as ROM, RAM, flash memory, and the like. The program instructions may include machine language code, such as that created by a compiler, as well as high-level language code that may be executed by a computer using an interpreter, etc.

Some aspects of the present invention have been described in the context of an apparatus, but it may also be described according to a corresponding method, wherein the block or apparatus corresponds to a step of the method or a feature of the step of the method. Similarly, the aspects described in the context of the method may also be represented as features of corresponding blocks or items or corresponding devices. Some or all of the steps of the method may be performed by (or using) hardware devices such as, for example, microprocessors, programmable computers, or electronic circuits. In some embodiments, one or more of the most important steps of the method may be performed by such a device.

While the present invention has been described above with reference to the exemplary embodiments, it may be understood by those skilled in the art that the present invention may be variously modified and changed without departing from the spirit and scope of the present invention disclosed in the claims.

**Claims**

1. An apparatus for determining thermal stability of a target material, the apparatus comprising:

    a memory; and

a processor executing at least one instruction in the memory,

wherein the at least one instruction comprises:

an instruction configured to acquire particle information constituting the target material;
an instruction configured to acquire a molecular structure using the particle information;
an instruction configured to acquire structural information from the molecular structure; and
an instruction configured to determine thermal stability of the target material using the structural information.

2. The apparatus of claim 1, wherein the instruction configured to acquire a molecular structure using the particle information comprises an instruction configured to acquire an energy-optimized molecular structure using the particle information.

3. The apparatus of claim 2, wherein the instruction configured to acquire the energy-optimized molecular structure using the particle information comprises an instruction configured to acquire an energy-optimized molecular structure using any one of first principles, semi-empirical, or empirical methods, based on the particle information.

4. The apparatus of claim 1, wherein the particle information comprises atom information and molecule information on the target material.

5. The apparatus of claim 1, wherein the particle information comprises a core moiety forming the target material and at least one functional group that is capable of being substituted to at least one derivative by bonding with the core moiety.

6. The apparatus of claim 1, wherein the structural information comprises at least one of radius of gyration (Rg) or asphericity (As).

7. The apparatus of claim 1, wherein the instruction configured to acquire the structural information from the molecular structure comprises:

an instruction configured to calculate a gyration tensor from the molecular structure; and
an instruction configured to calculate the structural information including at least one of radius of gyration or asphericity from the gyration tensor.

8. The apparatus of claim 7, wherein the gyration tensor is a matrix configured with a mass and position vectors of atoms.

9. The apparatus of claim 8, wherein the instruction configured to calculate the structural information comprises:

an instruction configured to diagonalize the gyration tensor to be expressed in terms of eigenvalues L1, L2, and L3;
an instruction configured to calculate the radius of gyration using the eigenvalues of the gyration tensor; and
an instruction configured to calculate the asphericity using the eigenvalues of the gyration tensor.

10. The apparatus of claim 1, wherein the instruction configured to determine thermal stability of the target material comprises an instruction configured to determine thermal stability of the target material by comparing the structural information to a threshold value.

11. The apparatus of claim 10, wherein the instruction configured to determine thermal stability of the target material by the comparing comprises:

an instruction configured to determine that thermal stability of the target material is high when radius of gyration included in the structural information exceeds a first threshold and asphericity included in the structural information exceeds a second threshold; and
an instruction configured to determine that thermal stability of the target material is low when the radius of gyration is equal to or less than the first threshold and the asphericity is equal to or less than the second threshold.

12. The apparatus of claim 1, wherein the instruction configured to determine thermal stability of the target material comprises an instruction configured to determine thermal stability of the target material by inputting the structural information to a pre-trained learning model.

13. The apparatus of claim 12, wherein the pre-trained learning model is a machine learning model using at least one experimental data according to a thermal stability experiment as training data.

14. The apparatus of claim 12, wherein the instruction configured to determine thermal stability of the target material further comprises an instruction configured to store result data of the determination of thermal stability in a database.

15. The apparatus of claim 14, wherein the instruction configured to determine thermal stability of the target material further comprises an instruction configured to retrain the learning model using at least one result data stored in the database.

16. The apparatus of claim 1, wherein the target material is applied to an organic thin film layer of an organic light emitting diode OLED.

17. A method of determining thermal stability for a target material, the method comprising:

acquiring particle information constituting the target material;
acquiring a molecular structure using the particle information;
acquiring structural information from the molecular structure; and
determining thermal stability of the target material using the structural information.

18. The method of claim 17, wherein the acquiring of the molecular structure using the particle information comprises acquiring an energy-optimized molecular structure using the particle information.

19. The method of claim 18, wherein the acquiring of the energy-optimized molecular structure using the particle information comprises acquiring an energy-optimized molecular structure using any one of first principles, semi-empirical, or empirical methods, based on the particle information.

20. The method of claim 17, wherein the particle information comprises atom information and molecule information on the target material.

21. The method of claim 17, wherein the particle information comprises a core moiety forming the target material and at least one functional group that is capable of being substituted to at least one derivative by bonding with the core moiety.

22. The method of claim 17, wherein the structural information comprises at least one of radius of gyration (Rg) or asphericity (As).

23. The method of claim 17, wherein the acquiring of the structural information from the molecular structure comprises:

calculating a gyration tensor from the molecular structure; and
calculating the structural information including at least one of radius of gyration or asphericity from the gyration tensor.

24. The method of claim 23, wherein the gyration tensor is a matrix configured with a mass and position vectors of atoms.

25. The method of claim 24, wherein the calculating of the structural information comprises:

diagonalizing the gyration tensor to be expressed in terms of eigenvalues L1, L2, and L3;
calculating the radius of gyration using the eigenvalues of the gyration tensor; and
calculating the asphericity using the eigenvalues of the gyration tensor.

26. The method of claim 17, wherein the determining of thermal stability of the target material comprises determining thermal stability of the target material by comparing the structural information to a threshold value.

27. The method of claim 26, wherein the determining of thermal stability of the target material by the comparing comprises:

determining that thermal stability of the target material is high when radius of gyration included in the structural information exceeds a first threshold and asphericity included in the structural information exceeds a second threshold; and
determining that thermal stability of the target material is low when the radius of gyration is equal to or less than the first threshold and the asphericity is equal to or less than the second threshold.

28. The method of claim 17, wherein the determining of thermal stability of the target material comprises determining thermal stability of the target material by inputting the structural information to a pre-trained learning model.

29. The method of claim 28, wherein the pre-trained learning model is a machine learning model using at least one experimental data according to a thermal stability experiment as training data.

30. The method of claim 28, wherein the determining of thermal stability of the target material further comprises storing result data of the determination of thermal stability in a database.

31. The method of claim 30, wherein the determining of thermal stability of the target material further comprises retraining the learning model using at least one result data stored in the database.

32. The method of claim 17, wherein the target material is applied to an organic thin film layer of an organic light emitting diode OLED.

[Figure 1]

[Figure 2]

```
                    ┌─────────────┐
                    │    START    │
                    └──────┬──────┘
                           │
           ┌───────────────▼─────────────────┐
           │ ACQUIRE PARTICLE INFORMATION    │───S1000
           │ ON TARGET MATERIAL              │
           └───────────────┬─────────────────┘
                           │
           ┌───────────────▼─────────────────┐
           │ ACQUIRE MOLECULAR STRUCTURE USING│───S3000
           │ PARTICLE INFORMATION            │
           └───────────────┬─────────────────┘
                           │
           ┌───────────────▼─────────────────┐
           │ ACQUIRE STRUCTURAL INFORMATION FROM│──S5000
           │ MOLECULAR STRUCTURE             │
           └───────────────┬─────────────────┘
                           │
           ┌───────────────▼─────────────────┐
           │ DETERMINE THERMAL STABILITY OF  │
           │ TARGET MATERIAL USING STRUCTURAL│───S7000
           │ INFORMATION                     │
           └───────────────┬─────────────────┘
                           │
                    ┌──────▼──────┐
                    │     END     │
                    └─────────────┘
```

[Figure 3]

Where, a, b = Functional groups; c = Core moiety

[Figure 4]

```
                    ┌─────────────┐
                    │    START    │
                    └─────────────┘
                           │
                           ▼
       ┌──────────────────────────────────────┐
       │  CALCULATE GYRATION TENSOR FROM       │───── S5100
       │  MOLECULAR STRUCTURE                  │
       └──────────────────────────────────────┘
                           │
   ┌ ─ ─ ─ ─ ─ ─ ─ ─ ─ ─ ─ │ ─ ─ ─ ─ ─ ─ ─ ─ ┐
                         CALCULATE STRUCTURAL
   │                     INFORMATION FROM       │───── S5500
                         GYRATION TENSOR
   │                       │                    │
                           ▼
   │   ┌──────────────────────────────────────┐│
       │  CALCULATE EIGENVALUES FROM GYRATION  │───── S5510
   │   │  TENSOR                               ││
       └──────────────────────────────────────┘
   │                       │                    │
                           ▼
   │   ┌──────────────────────────────────────┐│
       │  CALCULATE RADIUS OF GYRATION USING   │───── S5530
   │   │  EIGENVALUES                          ││
       └──────────────────────────────────────┘
   │                       │                    │
                           ▼
   │   ┌──────────────────────────────────────┐│
       │  CALCULATE ASPHERICITY USING          │───── S5550
   │   │  EIGENVALUES                          ││
       └──────────────────────────────────────┘
   └ ─ ─ ─ ─ ─ ─ ─ ─ ─ ─ ─ │ ─ ─ ─ ─ ─ ─ ─ ─ ┘
                           ▼
                    ┌─────────────┐
                    │     END     │
                    └─────────────┘
```

[Figure 5]

```
                    ┌─────────────┐
                    │    START    │
                    └─────────────┘
                           │
                           ▼
       ┌──────────────────────────────────────┐
       │  ACQUIRE MOLECULE INFORMATION ON TARGET│──── S7100
       │  MATERIAL BASED ON MACHINE LEARNING MODEL│
       └──────────────────────────────────────┘
                           │
                           ▼
       ┌──────────────────────────────────────┐
       │  STORE RESULT DATA AND UPDATE MACHINE  │──── S7500
       │  LEARNING MODEL                        │
       └──────────────────────────────────────┘
                           │
                           ▼
                    ┌─────────────┐
                    │     END     │
                    └─────────────┘
```

[Figure 6]

EP 4 439 568 A1

16

A: ORGANIC THIN FILM FORMATION AREA OF A MATERIAL
B: THERMAL STABILITY DETERMINATION AREA (A MATERIAL DETECTION)
C: PARTITION WALL (BANK) SEPARATING A AND B

[Figure 7]

[Figure 8]

Core

Functional groups

[Figure 9]

[Figure 10]

[Figure 11]

<table>
<tr><td colspan="2">**INTERNATIONAL SEARCH REPORT**</td><td>International application No.<br><br>**PCT/KR2023/010805**</td></tr>
</table>

| A. | CLASSIFICATION OF SUBJECT MATTER |
|---|---|

**G16C 20/20**(2019.01)i; **G16C 20/70**(2019.01)i; **H10K 85/00**(2023.01)i

According to International Patent Classification (IPC) or to both national classification and IPC

| B. | FIELDS SEARCHED |
|---|---|

Minimum documentation searched (classification system followed by classification symbols)

G16C 20/20(2019.01); G01N 27/62(2006.01); G06F 17/50(2006.01); G06N 20/00(2019.01); G06N 3/04(2006.01); G16C 20/30(2019.01); H01J 49/26(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 열적 안정성(thermal stability), 프로세서(processor), 회전텐서(Gyration tensor), 기계학습(Machine learning), 유기발광소자(Organic light-emitting diodes)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 111785332 A (CHEMICAL REGISTRATION CENTER OF EMERGENCY MANAGEMENT DEPARTMENT) 16 October 2020 (2020-10-16)<br>See claim 1. | 1-32 |
| Y | KR 10-2284532 B1 (STANDIGM INC.) 03 August 2021 (2021-08-03)<br>See claims 1, 2, 7-9 and 13; and paragraph [0040]. | 1-32 |
| A | ZHAO, Y. et al. Data-driven machine learning models for the quick and accurate prediction of thermal stability properties of OLED materials. Materials Today Chemistry. 2021, vol. 22, document no. 100625, pp. 1-29.<br>See entire document. | 1-32 |
| A | JP 2004-028782 A (HITACHI LTD.) 29 January 2004 (2004-01-29)<br>See entire document. | 1-32 |

| ✓ | Further documents are listed in the continuation of Box C. | ✓ | See patent family annex. |
|---|---|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **23 November 2023** | **23 November 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

| | INTERNATIONAL SEARCH REPORT | International application No. |
| | | PCT/KR2023/010805 |

**C.     DOCUMENTS CONSIDERED TO BE RELEVANT**

| Category* | Citation of document, with indication, where appropriate, of the relevant passages | Relevant to claim No. |
|---|---|---|
| A | KR 10-2019-0129522 A (KOREA INSTITUTE OF SCIENCE AND TECHNOLOGY) 20 November 2019 (2019-11-20)<br>     See entire document. | 1-32 |

<table>
<tr><td colspan="3">INTERNATIONAL SEARCH REPORT</td><td>International application No.</td></tr>
<tr><td colspan="3">Information on patent family members</td><td>**PCT/KR2023/010805**</td></tr>
</table>

| Patent document cited in search report | | | Publication date (day/month/year) | Patent family member(s) | | | Publication date (day/month/year) |
|---|---|---|---|---|---|---|---|
| CN | 111785332 | A | 16 October 2020 | None | | | |
| KR | 10-2284532 | B1 | 03 August 2021 | None | | | |
| JP | 2004-028782 | A | 29 January 2004 | EP | 1376651 | A2 | 02 January 2004 |
| | | | | EP | 1376651 | A3 | 15 February 2006 |
| | | | | EP | 1376651 | B1 | 11 June 2014 |
| | | | | EP | 1376651 | B8 | 16 July 2014 |
| | | | | JP | 3743717 | B2 | 08 February 2006 |
| | | | | US | 2003-0236634 | A1 | 25 December 2003 |
| | | | | US | 2003-0236636 | A1 | 25 December 2003 |
| | | | | US | 2005-0139761 | A1 | 30 June 2005 |
| | | | | US | 6745134 | B2 | 01 June 2004 |
| | | | | US | 6907352 | B2 | 14 June 2005 |
| | | | | US | 7158893 | B2 | 02 January 2007 |
| KR | 10-2019-0129522 | A | 20 November 2019 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220133170